(19) Europäisches Patentamt
European Patent Office
Office européen des brevets

(11) **EP 4 112 735 A1**

(12) **EUROPEAN PATENT APPLICATION**
published in accordance with Art. 153(4) EPC

(43) Date of publication:
**04.01.2023 Bulletin 2023/01**

(21) Application number: **21761452.8**

(22) Date of filing: **26.02.2021**

(51) International Patent Classification (IPC):
*C12Q 1/34* (2006.01)     *C12Q 1/6851* (2018.01)
*C12Q 1/686* (2018.01)     *C12Q 1/70* (2006.01)
*G01N 33/569* (2006.01)

(52) Cooperative Patent Classification (CPC):
**C12Q 1/34; C12Q 1/6851; C12Q 1/686; C12Q 1/70;
G01N 33/569**

(86) International application number:
**PCT/JP2021/007566**

(87) International publication number:
**WO 2021/172573 (02.09.2021 Gazette 2021/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.02.2020  JP 2020033114**

(71) Applicant: **Asahi Kasei Medical Co., Ltd.
Tokyo 100-0006 (JP)**

(72) Inventors:
• **KAYUKAWA, Taiki
Tokyo 100-0006 (JP)**
• **YANAGIDA, Koichiro
Tokyo 100-0006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **METHOD FOR EVALUATING VIRAL CLEARANCE PERFORMANCE**

(57)     Provided is a method for evaluating the viral clearance capability of a virus removal medium, comprising the steps of: (a) adding a viral capsid-containing liquid to a solution to be purified; (b) contacting the virus removal medium with the solution to be purified that has been supplemented with the viral capsid-containing liquid to harvest a purified solution; and (c) quantifying a total viral capsid in the solution to be purified before the purification and a total viral capsid in the purified solution.

**Fig. 1**

EP 4 112 735 A1

**Description**

Technical Field

The present invention relates to a method for evaluating the viral clearance capability of a virus removal membrane.

Background Art

Pharmaceutical formulations containing biological materials include fractionated plasma products which are purified from blood, and biological products which are produced by biotechnology. These pharmaceutical formulations containing biological materials have the risk of being contaminated with viruses. In general, the pharmaceutical formulations containing biological materials need to insure safety against viruses. Hence, the production of the pharmaceutical formulations containing biological materials needs to involve the step of sufficiently removing or inactivating viruses that are contained or may be contained in the pharmaceutical formulations containing biological materials, i.e., a virus removal step (see, for example, Non Patent Literature 1). The ability of the virus removal step to remove or inactivate viruses is evaluated and/or studied in a scaled down system before actual production of the pharmaceutical formulations containing biological materials. Such evaluation and/or study is referred to as a viral clearance test.

In the process of producing pharmaceutical formulations containing biological materials, low-pH treatment or a treatment step with a virus removal medium is regarded as a robust virus removal step (see, for example, Non Patent Literature 2). The treatment step with a virus removal medium such as a virus removal membrane is prominent because this step can be carried out on all viruses, irrespective of the presence or absence of envelopes.

The viral clearance test in the process of producing pharmaceutical formulations containing biological materials usually involves evaluating viral clearance capability by measuring viral concentrations before and after an arbitrary production step, and calculating, for example, a logarithmic reduction rate (LRV) thereof (see, for example, Non Patent Literature 1). In this respect, the viral clearance capability of a virus removal step is evaluated by adding a virus-containing liquid (virus spiking) to a biological material-containing solution before the virus removal step, and measuring viral concentrations before and after contact of the biological material-containing solution with a virus removal medium.

The types of viruses for use in evaluating viral clearance capability are described as a guideline in q5a of ICH (International Conference on Harmonization of Technical Requirements for Registration of Pharmaceuticals for Human Use), which mentions "animal parvovirus" as an example of useful nonspecific model viruses (see, for example, Non Patent Literature 1). Among others, minute virus of mice (MVM) and porcine parvovirus (PPV) are particularly used with high frequency in the viral clearance test of pharmaceutical formulations containing biological materials among many viral clearance tests cited in ICH-q5a. An infectivity measurement method including assay based on an endpoint method or a local lesion count method, or quantitative PCR (quantitative polymerase chain reaction: qPCR) is typically used as a method for measuring viral concentrations thereof (see, for example, Non Patent Literatures 2 and 3).

Citation List

Patent Literature

Patent Literature 1: International Publication No. WO 2014/080676A1
Patent Literature 2: U.S. Patent Application Publication No. 2012/0088228
Patent Literature 3: Japanese Patent No. 4024041

Non Patent Literature

Non Patent Literature 1: Viral Safety Evaluation of Biotechnology Product Derived from Cell Lines of Human or Animal Origin Q5A(R1)
Non Patent Literature 2: Note For Guidance On Virus Validation Studies: The Design, Contribution And Interpretation Of Studies Validating The Inactivation and Removal Of Viruses
Non Patent Literature 3: Guideline on safety assurance of fractionated plasma products against viruses (Notification No. 1047 dated August 30, 1999, Pharmaceutical and Medical Safety Bureau, Ministry of Health, Labour and Welfare, Japan)

Non Patent Literature 4: Raphael Wolfisberg et al., Journal of Virology (2016)
Non Patent Literature 5: Beatriz Maroto et al., JOURNAL OF VIROLOGY (2004)
Non Patent Literature 6: Uirusu Jikkengaku Kakuron (Virology Experiments in Detail in English), Maruzen Publishing Co., Ltd., edited by Student's Association of National Institute of Health, pp. 22-23
Non Patent Literature 7: V. Hutornojs et al., (2012) Env, Exp. Biol. 10: pp. 117-123
Non Patent Literature 8: Anthony M. D'Abramo Jr. et al., 2005 Virology
Non Patent Literature 9: Joshua C Grieger et al., Molecular Therapy 2015
Non Patent Literature 10: Pavel Plevka et al., JOURNAL OF VIROLOGY (2011)
Non Patent Literature 11: PETER TATTERSALL et al., JOURNAL OF VIROLOGY (1976)

Summary of Invention

Technical Problem

An object of the present invention is to provide a method capable of more accurately evaluating the viral clearance capability of a virus removal medium in the step of removing a virus in a solution.

Solution to Problem

According to the findings of the present inventors, a virus-containing liquid for use in virus spiking may contain two types of viruses, i.e., a virus that has a nucleic acid and is infectious and a virus that has a nucleic acid but is not infectious. The virus-containing liquid may further contain empty viral particles which neither have a nucleic acid nor are infectious (see, for example, Non Patent Literatures 4 and 5). The empty viral particles are also called empty capsids or virus-like particles. The term "virus that is not infectious" or "non-infectious virus" refers to a virus that may have the ability to invade cells and the ability to proliferate in the cells and however, neither causes virus release associated with cytopathic effects and cell death in host cells after entry nor is detected by an infectivity measurement method. All of the infectious virus, the virus that is not infectious, and the empty viral particles are viral capsids. In the present disclosure, a liquid containing such a virus and/or empty viral particles is referred to as a viral capsid-containing liquid.

The infectivity measurement method can detect the infectious virus and however, cannot detect the virus that is not infectious and the empty viral particles. Quantitative PCR can detect the virus that has a nucleic acid and is infectious and the virus that has a nucleic acid but is not infectious, and however, cannot detect the empty viral particles having no nucleic acid. The present inventors have found for the first time that the viral capsid-containing liquid has a virus or empty viral particles that cannot be quantified by a conventional method for evaluating viral clearance capability; and that in the presence of the virus or the empty viral particles, there arises the serious issue that viral clearance capability evaluation is affected thereby, i.e., the ability of a virus removal medium to remove or inactivate viruses is underestimated or is not correctly evaluated.

The present inventors have found that, for example, in the case of using a viral capsid-containing liquid that contains empty viral particles, the empty viral particles, which cannot be detected by quantitative PCR, hasten the breakthrough of a virus removal medium so that a virus having a nucleic acid which is supposed to be measured leaks out of the virus removal medium earlier as compared with the case where the medium is loaded with only the virus having a nucleic acid, disadvantageously resulting in underestimated viral clearance capability of the virus removal medium. As a result of conducting diligent studies, the present inventors have found that the viral clearance capability of a virus removal medium may be able to be more accurately evaluated by using a viral capsid-containing liquid from which empty viral particles have been removed.

The present inventors have further found that in the case of using a viral capsid-containing liquid that contains a virus that has a nucleic acid but is not infectious and/or empty viral particles in an infectivity measurement method, the virus that has a nucleic acid but is not infectious and/or the empty viral particles hasten the breakthrough of a virus removal medium so that an infectious virus which is supposed to be measured leaks out of the virus removal medium early, disadvantageously resulting in underestimated viral clearance capability of the virus removal medium. As a result of conducting diligent studies, the present inventors have found that viral clearance capability may be able to be more accurately evaluated by determining beforehand the ratio of the number of total viral capsid particles to infectivity (number of total viral capsid particles /infectivity) in the viral capsid-containing liquid used, and recalculating results of viral clearance evaluation by an infectivity measurement method as results of evaluation based on the number of the total viral capsid particles including a virus that has a nucleic acid but is not infectious and empty viral particles. In the present disclosure, the term "total viral capsid" refers to all types of viral capsids present in a viral capsid-containing liquid. In the present disclosure, the term "number of total viral capsid particles" refers to the number of particles of all types of viral capsids present in a viral capsid-containing liquid. The present inventors have found that in conventional techniques, the viral clearance capability of a virus removal medium is underestimated because all types of viral capsids used in viral clearance evaluation are not quantified.

Thus, the present inventors have completed the present invention by finding that in viral clearance capability evaluation, the viral clearance capability of a virus removal medium can be more accurately evaluated by appropriately selecting the viral capsid-containing liquid used depending on the type of a viral quantification method or appropriately selecting a quantification method depending on the viral capsid-containing liquid used in evaluating the viral clearance capability of the virus removal medium, and detecting a total viral capsid in a solution before and after purification with the virus removal medium, as compared with conventional techniques which do not involve selecting a viral capsid-containing liquid depending on the quantification method used.

Specifically, the present invention is as follows.

[1] A method for evaluating the viral clearance capability of a virus removal medium, comprising the steps of:

> (a) adding a viral capsid-containing liquid to a solution to be purified;
> (b) contacting the virus removal medium with the solution to be purified that has been supplemented with the viral capsid-containing liquid to harvest a purified solution; and
> (c) quantifying the number of total viral capsid in the solution to be purified before the purification and the number of total viral capsid in the purified solution.

[2] The method according to [1], wherein in the step (b), the solution to be purified that has been supplemented with the viral capsid-containing liquid is passed through the virus removal medium to harvest the solution purified.

[3] The method according to [1] or [2], wherein

> empty viral particles have been removed from the viral capsid-containing liquid to be added to the solution to be purified, and
> in the step (c), a nucleic acid of a virus in the solution to be purified before the purification and a nucleic acid of a virus in the purified solution are quantified.

[4] The method according to [3], wherein the empty viral particles are removed by centrifugation.

[5] The method according to [3] or [4], wherein the empty viral particles are removed by density gradient ultracentrifugation.

[6] The method according to any of [3] to [5], wherein the empty viral particles are removed by density gradient ultracentrifugation using iodixanol.

[7] The method according to any of [3] to [6], wherein the nucleic acid is quantified by quantitative PCR or fluorescence flow cytometry.

[8] The method according to any of [3] to [7], wherein the nucleic acid is quantified by quantitative PCR.

[9] The method according to any of [1] to [8], wherein the virus removal medium is a medium having a form of a membrane or beads.

[10] The method according to any of [1] to [9], wherein the virus removal medium is a virus removal membrane.

[11] The method according to any of [3] to [8], further comprising the step of removing a free nucleic acid in the solution before the step of quantifying the nucleic acid of the virus.

[12] The method according to [11], wherein the free nucleic acid is removed with nuclease.

[13] The method according to [12], wherein the nuclease is DNaseI or Benzonase.

[14] The method according to any of [1] to [13], wherein the virus is a virus belonging to *Parvoviridae*.

[15] The method according to any of [1] to [14], wherein the viral capsid-containing liquid is a viral capsid-containing liquid that contains a naturally derived viral capsid.

[16] The method according to any of [1] to [15], wherein the viral capsid-containing liquid contains viral particles having a nucleic acid, and empty viral particles.

Advantageous Effects of Invention

The present invention can provide a method capable of more accurately evaluating the viral clearance capability of a virus removal medium in the step of removing a virus in a solution.

Brief Description of Drawing

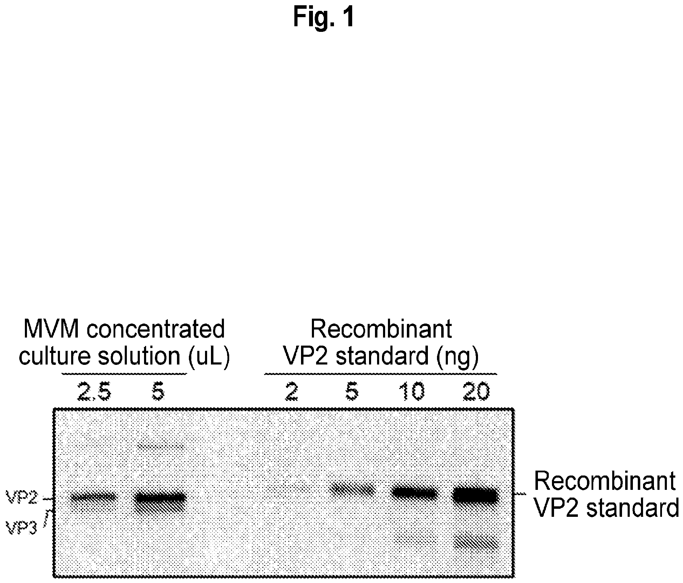

[Figure 1] Figure 1 shows results of blotting of a concentrated MVM culture solution and a recombinant MVM-VP2 standard. The numerical values indicated on lanes depict the amount of sample load ($\mu$L) of the concentrated MVM culture solution and the amount of sample load ($\mu$L) of the recombinant VP2 standard, respectively, in SDS-PAGE.

Description of Embodiments

Hereinafter, the mode for carrying out the present invention (hereinafter, also referred to as the "present embodiment") will be described. The present invention is not limited by embodiments given below and can be carried out by making various changes or modifications therein without departing from the spirit of the present invention. The embodiments given below illustrate methods, etc. for specifying the technical idea of this invention, and the present invention is not limited by these examples.

A method for evaluating a viral clearance capability of a virus removal medium according to an embodiment comprises the steps of: (a) adding a viral capsid-containing liquid to a solution to be purified; (b) contacting the virus removal medium with the solution to be purified that has been supplemented with the viral capsid-containing liquid to harvest a purified solution; and (c) quantifying the number of total viral capsid in the solution to be purified before the purification and the number of total viral capsid in the purified solution.

In one embodiment, examples of the virus include an infectious virus and a non-infectious virus. Preferably, the virus is naturally derived. There also exist empty viral particles which neither are virally infectious nor have a nucleic acid (viral nucleic acid).

In one embodiment, the empty viral particles are viral capsids that neither are infectious nor have a nucleic acid. The empty viral particles also include particles incorporating a very small nucleic acid fragment that does not influence a buoyant density. Preferably, the empty viral particles are naturally derived.

In one embodiment, the viral capsid includes a virus and/or empty viral particles. Preferably, the viral capsid is naturally derived.

The naturally derived virus and the naturally derived empty viral particles are distinguished from a virus synthetically produced by an approach typified by recombinant expression or chemical synthesis. Examples of the naturally derived virus and the naturally derived empty viral particles respectively include a virus and empty viral particles obtained as a result of culturing host cells infected with a virus in a culture medium, and a virus and empty viral particles obtained by transfecting cultured cells with a viral nucleic acid, followed by culture.

In one embodiment, the viral capsid-containing liquid is not particularly limited as long as the liquid contains a virus and/or empty viral particles. Examples thereof include a liquid containing a naturally derived virus and/or naturally derived empty viral particles. Examples of the viral capsid-containing liquid include a liquid containing at least one of three viral capsids, an infectious virus, a non-infectious virus, and empty viral particles. The viral capsid-containing liquid preferably contains at least an infectious virus or a non-infectious virus. A liquid containing only empty viral particles among the three viral capsids is also one example of the viral capsid-containing liquid. A culture solution of a virus obtained as a result of culturing host cells infected with a virus in a culture medium is also one example of the viral capsid-containing liquid. The virus may exist in an infected state in host cells or may exist in a free state in the viral capsid-containing liquid (e.g., a culture solution of a virus). The viral capsid-containing liquid may be a culture supernatant of cultures obtained by infecting host cells with a virus, followed by culture, and/or a disrupted product of infected cells (hereinafter, also simply referred to as an "infected cell disruption"). Examples of the "culture supernatant" include a culture medium after infection and culture. Examples of the "infected cell disruption" include a so-called lysate obtained by disrupting cells after infection and culture through freezing-thawing or homogenization. A mixed liquid of a plurality of viral capsid-containing liquids is also one example of the viral capsid-containing liquid.

In one embodiment, the viral capsid-containing liquid is not particularly limited as long as the liquid is the viral capsid-containing liquid described above. Examples thereof include a solution, a suspension, and a gel-like liquid. A solution is a preferred form. Examples of the solution include a culture solution of a virus, and a liquid obtained by purifying a culture solution of a virus for the removal of contents other than a viral capsid.

The infectious virus and the non-infectious virus have a nucleic acid (viral nucleic acid). The infectious virus and the non-infectious virus are also collectively referred to as a "virus having a nucleic acid", a "full capsid virus" or a "FC virus". On the other hand, the empty viral particles are a viral capsid that neither is infectious nor has a nucleic acid (viral nucleic acid). The empty viral particles are also referred to as a "virus having no nucleic acid". The three viral capsids, i.e., the infectious virus, the non-infectious virus, and the empty viral particles, which may be contained in the viral capsid-containing liquid are collectively referred to as a total viral capsid. However, when the empty viral particles have been removed from the viral capsid-containing liquid, the infectious virus and the non-infectious virus which may be contained in the viral capsid-containing liquid are collectively referred to as a total viral capsid.

In one embodiment, the virus is not particularly limited as long as the virus is contained or may be contained in a biological material. Examples thereof include minute virus of mice, porcine parvovirus, reovirus type 3, poliovirus, pseudorabies virus, human herpes virus 1, xenotropic murine leukemia virus, and bovine viral diarrhea virus. Minute virus of mice and porcine parvovirus are preferred forms, and minute virus of mice is a more preferred form.

In one embodiment, when the virus is parvovirus, examples thereof include a virus having an average particle size of 20 nm or larger and 40 nm or smaller. Specific examples thereof include porcine parvovirus (PPV) and minute virus of mice (MVM). In the present embodiment, a "culture supernatant" and/or an "infected cell disruption" obtained by infecting host cells with arbitrary parvovirus, followed by culture is also referred to as a "viral culture solution".

In one embodiment, the viral capsid-containing liquid can be produced, for example, as described below. In order to produce the viral capsid-containing liquid, host cells for a virus are first cultured. Any cell that is infected with a virus and proliferates can be used as the host cells. When the virus is parvovirus, any cell that is infected with the parvovirus and proliferates can be used. When the virus is porcine parvovirus, for example, PK-13 cells (ATCC) and PK-15 cells (ATCC) can be used. When the virus is minute virus of mice, for example, mouse fibroblasts A9, simian virus 40-transformed human fibroblasts (324K cells, NB324K cells, etc.), and Chinese hamster ovary cells (CHO) can be used.

In one embodiment, the method for culturing the host cells is not limited. Any method can be used, such as a method involving use of a culture flask, a method including fixing cells to a microcarrier and then stirring culture, a method involving use of a roller bottle, or a method involving use of a bioreactor. Any of adhesion culture and suspension culture can be used.

In one embodiment, examples of the culture flask include a commercially available flask for tissue culture (e.g., manufactured by Falcon/Corning Inc.) and a multiwell plate.

In one embodiment, examples of the microcarrier include a commercially available microcarrier, for example, Cytodex and Cytopore manufactured by GE Healthcare Japan Corp.

In one embodiment, examples of the roller bottle include a commercially available roller bottle, for example, CELLMASTER cell culture roller bottle manufactured by Greiner Bio-One International GmbH.

In any of the culture methods, the culture temperature may be, for example, 33°C or higher and 39°C or lower, which are generally applied to mammalian cell culture. Particularly, 37°C is preferred. The pH of the culture medium is preferably 7 or higher and 8 or lower, which are generally applied to mammalian cell culture. The concentration of the culture medium is preferably a concentration isotonic (having an osmotic pressure equal) to host cells.

In one embodiment, examples of the culture medium include DMEM medium, MEM medium, CD medium, SFM medium, OptiPro SMF medium, and Viral Vaccine Platform medium (SFM4Mega Vir medium, Vaccine Xpress medium, and CDM4 Avian medium). These culture media can be obtained from Gibco, Thermo Fisher Scientific Inc., GE Healthcare Japan Corp., and the like.

The cultured host cells in an uninfected state are infected with seeds of a virus. The seed virus used can be obtained by increasing in number a virus purchased from ATCC or the like, for example, by a method described in the present specification, and dividing the resulting viruses into small portions in cryotubes or the like, followed by cryopreservation at -80°C. Examples of the purchasable seed virus include VR-742 (ATCC) when the virus is PPV, and VR-1346 (ATCC) when the virus is MVM.

In the infection method with the seed virus, a predetermined amount of the virus (in a state suspended in a culture medium) can be added into a culture vessel containing the cultured host cells (in uninfected state).

For a smaller amount of impurities other than parvovirus in the culture solution, the culture medium is desirably a serum-free medium which contains no serum. However, efficient proliferation of a virus can be achieved by culture in a culture medium containing serum such as fetal bovine serum (FBS). Hence, the culture medium may be changed from a serum-containing medium to a serum-free medium 24 hours or longer before harvesting the viral culture solution, for example, by a method described in Patent Literature 1 so that a virus can be produced from the cells into the culture medium containing no impurities derived from serum, and harvested therefrom. Before a subsequent ultracentrifugation step, low-speed centrifugation and sterilization membrane filtration may be performed in order to remove foreign matter having a large particle size, such as cell debris, present in the parvoviral culture solution. The low-speed centrifugation can be carried out by selecting an acceleration of gravity at which a virus is not precipitated whereas cell debris is precipitated. For example, centrifugation at 3,000 rpm for 20 minutes (see, for example, Patent Literature 1) is performed, though the centrifugation is not limited by these conditions. The sterilization membrane filtration can employ a membrane having a pore size of 0.45 $\mu$m, 0.22 $\mu$m or 0.1 $\mu$m. Examples of the membrane having such a pore size include Bottle Top Filter 0.45 $\mu$m manufactured by Thermo Fisher Scientific Inc. and Bottle Top Filter 0.22 $\mu$m manufactured by Thermo Fisher Scientific Inc.

In order to increase the concentration of the viral capsid-containing liquid to be subjected to a test and improve the purity thereof, the harvested viral culture solution may be concentrated and purified. A known method can be used as a concentration method. For example, any of many known methods can be used, such as a precipitation method including salting out using $CsCl_2$, a PEG precipitation method, a method involving use of an ultrafiltration membrane, or an ultracentrifugation method (see, for example, Patent Literatures 1 and 2, and Non Patent Literature 6). Among them, a concentration method based on an ultracentrifugation method or a PEG precipitation method is preferred because of convenient solvent replacement and a high rate of virus harvest.

In the case of using, for example, an ultracentrifugation method, ultracentrifugation is performed using a relative centrifugal force (g) and a time which precipitate a virus. After precipitation of the virus, the supernatant is removed, and the precipitated fraction is resuspended in a proper solvent. When the virus is parvovirus, ultracentrifugation conditions for precipitation thereof are known and these conditions can be used (see, for example, Non Patent Literature 7). In the case of using, for example, Optima L-90K ultracentrifuge and Type 45 Ti rotor manufactured by Beckman Coulter Inc., parvovirus is precipitated by ultracentrifugation at 29400 rpm for 2 hours. For example, a TNE (Tris buffer solution + NaCl + EDTA) buffer solution or a PBS buffer solution generally used can be used as a resuspending solvent. The solvent is preferably a PBS buffer solution.

In the case of using a PEG precipitation method, centrifugation is performed by the addition of polyethylene glycol and a salt to some extent that a virus is condensed. After precipitation of the virus, the supernatant is removed, and the precipitated fraction is resuspended in a proper solvent. In this respect, experimental conditions generally used for virus precipitation can be exploited (see, for example, Patent Literature 1). For example, PEG6000 can be used as polyethylene glycol. When the virus is parvovirus, 10.0 w/v% (final concentration) PEG6000 and 0.1 mol/L (final concentration) sodium chloride are added thereto and the mixture is stirred at 4°C for 2 hours, followed by centrifugation at $10.000 \times g$ for 30 minutes to precipitate parvovirus. For example, a TNE (Tris buffer solution + NaCl + EDTA) buffer solution or a PBS buffer solution generally used can be used as a resuspending solvent. A PBS buffer solution is preferred.

Preferably, empty viral particles have been removed from the viral capsid-containing liquid to be added to the solution to be purified. The removal of empty viral particles from the viral capsid-containing liquid enables the viral clearance capability of a virus removal medium to be accurately evaluated without being influenced by the amount of the empty viral particles contained in the viral capsid-containing liquid. The viral capsid-containing liquid from which empty viral particles have been removed can be produced, for example, as follows.

A method including separating or removing empty viral particles from a viral culture solution can be used as a method for preparing the viral capsid-containing liquid from which empty viral particles have been removed. A method based on density gradient ultracentrifugation or a method based on ion-exchange chromatography can be used as a method for separating or removing empty viral particles. A method based on density gradient ultracentrifugation is preferred.

In the density gradient ultracentrifugation method, empty viral particles are separated through the use of the difference in buoyant density between a virus having a nucleic acid and the empty viral particles having no nucleic acid. The conditions of this ultracentrifugation are not limited to specific conditions. There are some reports on the ultracentrifugation conditions for the separation of an infectious virus and a non-infectious virus from empty viral particles, and these reports can be used (see, for example, Non Patent Literatures 4 and 8) .

For example, 1 mL of the viral culture solution can be loaded to the uppermost part of a noncontinuous density gradient (55%-2 mL, 45%-2 mL, 40%-2 mL, 35%-2 mL, 25%-1.5 mL, 15%-1.5 mL) of an aqueous iodixanol (manufactured by Axis-Shield Diagnostics Ltd., Optiprep) solution with PBS-1 mmol/L $MgCl_2$-2.5 mmol/L KCl buffer solution (pH 7.2) as a solvent formed in a UC tube (13.2 mL, #344059 manufactured by Beckman Coulter Inc.), and ultracentrifuged at 35000 rpm at 4°C for 18 hours using an ultracentrifuge (Optima L-90K manufactured by Beckman Coulter Inc.) and SW41 rotor to separate a virus having a nucleic acid from empty viral particles. Only a fraction that corresponds to the buoyant density of the virus having a nucleic acid is harvested from the sample thus ultracentrifuged to remove the empty viral particles from the viral culture solution. When the virus is parvovirus, the buoyant density of the empty viral particles is 1.1 g/mL or more and 1.2 g/mL or less and the buoyant density of the virus having a nucleic acid is 1.2 g/mL or more and 1.3 g/mL or less. Preferably, this ultracentrifugation can be repetitively performed a total of two times, thereby enhancing the degree of separation of empty viral particles.

Since the fraction containing the virus having a nucleic acid obtained by ultracentrifugation is a high concentration of an aqueous iodixanol solution, it is preferred to decrease the iodixanol concentration by solvent replacement. A method for this solvent replacement can be selected from among known solvent replacement methods and carried out. Examples thereof include desalting, UF membrane filtration, and dialysis. Desalting is preferred. Desalting using Zeba Spin Desalting Columns 40K MWCO (Thermo Fisher Scientific Inc.) is more preferred.

It can be confirmed by observation under a transmission electron microscope (TEM) that the empty viral particles have been removed from the viral capsid-containing liquid. Examples of the observation method involving use of TEM include a negative staining observation method involving use of 1.0% uranyl acetate, and a cryo-electron microscopy (CryoEM) observation method. In the negative staining observation method, the virus having a nucleic acid is observed as white particles while the empty viral particles which are a viral capsid having no nucleic acid are observed as black particles. In the CryoEM method, the virus having a nucleic acid is observed as black particles while the empty viral particles are observed as white particles. Therefore, these particles can be visually discriminated from each other (see, for example, Non Patent Literature 9) .

For example, in the case of measuring the percent content of empty viral particles based on a total viral capsid, a portion of the viral capsid-containing liquid to be measured is collected and observed under TEM. A virus having a nucleic acid and empty viral particles are counted per field of view, and the ratio of the number of the empty viral particles to the number of the total viral capsid particles is calculated. A mean of results of determining this ratio in three fields of view can be regarded as the percent content of the empty viral particles in the viral capsid-containing liquid to be measured. In one embodiment, examples of the percent content of the empty viral particles based on the total viral capsid in the viral capsid-containing liquid from which the empty viral particles have been removed include, but are not particularly limited to, 50% or less, 44% or less, 38% or less, 30% or less, 20% or less, and 10% or less in terms of the upper limit. The percent content is preferably 38% or less, more preferably 10% or less. The lower limit is preferably no empty viral particle contained (0%) or equal to or lower than the detection limit of the measurement method involving transmission electron microscope observation. Examples thereof include 0.5% or more, 1% or more, 2% or more, 5% or more, and 10% or more. Preferably, the viral capsid-containing liquid from which the empty viral particles have been removed is substantially free of the empty viral particles. The viral capsid-containing liquid may be contaminated with components other than viruses. Such a viral capsid-containing liquid is not excluded from the present invention. In another embodiment, the viral capsid-containing liquid may be substantially free of components other than viruses.

In one embodiment, the biological material contained in the solution to be purified by filtration through a virus removal medium is not particularly limited as long as a virus contained, together with the material, in the solution is to be removed. Examples thereof include a protein. Examples of the protein include albumin, globulin, and fibrinogen. In another embodiment, examples of the protein include an antibody. The biological material also includes biologics or fractionated plasma products. The biological material may be obtained as a commercially available product or may be produced by a conventional method known in the art.

In one embodiment, a biological material-containing solution serving as the solution to be purified is not particularly limited as long as the solution contains a biological material. Examples of the solvent in the biological material-containing solution include an aqueous solution, a phosphate buffer solution, a sodium acetate buffer solution, and a Tris buffer solution. An aqueous solution is a preferred form.

In one embodiment, the pH of the biological material-containing solution may be, for example, 12 or lower, 10 or lower, 9 or lower, 8 or lower, and 5 or lower, in terms of the upper limit. It may be, for example, 2 or higher, 3 or higher, and 4 or higher, in terms of the lower limit.

In one embodiment, the biological material concentration (mg/mL) of the biological material-containing solution may be, for example, 200 mg/mL or lower, 100 mg/mL or lower, and 10 mg/mL or lower, in terms of the upper limit. It may be, for example, higher than 0 mg/mL, 5 mg/mL or higher, 10 mg/mL or higher, and 20 mg/mL or higher, in terms of the lower limit.

In one embodiment, the virus removal medium is not particularly limited by its form as long as the medium can remove a virus. Examples of the form include beads, a gel, a sponge, and a powder. In another embodiment, examples thereof include a fiber and a membrane (a flat membrane and a hollow fiber membrane; a so-called virus removal membrane). In still another embodiment, the form of the virus removal medium is a flat membrane or a hollow fiber membrane. In a yet still another embodiment, the form is a flat membrane. In a yet still another embodiment, the form is a hollow fiber membrane. In a further alternative aspect, the virus removal medium is in the form of a membrane or beads. The virus removal medium may be a porous compact. The porous compact may be a microporous membrane, a nonwoven fabric, a woven fabric, a monolith, a gel, or a particle bed.

In one embodiment, the material of the virus removal medium is not particularly limited as long as the material can remove a virus. Examples thereof include polyethersulfone, polysulfone, polyvinylidene fluoride, cellulose, a cellulose derivative, and a mixture thereof. The virus removal medium made of each of these materials can be produced by a method known in the art.

In one embodiment, the virus removal medium is preferably a virus removal membrane. The virus removal membrane can be appropriately selected depending on the size of the virus used, and is not particularly limited as long as the virus removal membrane can remove the virus. Examples thereof include a membrane having LRV of 4 or more for parvovirus. The filtration membrane having LRV of 4 or more for parvovirus is a membrane having LRV of 4 or more when the filtration membrane used is loaded with 50 L/m$^2$ of a solution containing parvovirus PPV or MVM. A 10 mg/mL human IgG solution (PBS buffer, pH 7.4) adjusted to a parvovirus PPV or MVM concentration of 6.5 given by $Log_{10}$ [$TCID_{50}$/mL] is used as the solution containing parvovirus. The average pore size of the virus removal membrane is not particularly limited as long as the average pore size is equal to or smaller than the size of the virus used. In the case of using, for example, parvovirus, the average pore size of the virus removal membrane is, for example, in the range of 5 nm or larger and 24 nm or smaller, preferably in the range of 14 nm or larger and 22 nm or smaller, more preferably 18 nm or larger and 20 nm or smaller. Specific examples thereof include Planova (manufactured by Asahi Kasei Medical Co., Ltd.), Viresolve Pro (manufactured by Merck KGaA), Pegasus Prime and Pegasus SV4 (manufactured by Pall Corp.), and VirosartCPV (manufactured by Sartorius AG).

In one embodiment, when the virus removal medium is a virus removal membrane, the virus removal membrane is not particularly limited as long as the virus removal membrane can substantially remove a virus. One example thereof is a virus removal membrane having a nominal pore size of approximately 15 nm or larger and approximately 75 nm or smaller. For example, a membrane having a nominal pore size of 20 nm is capable of removing, for example, parvovirus (diameter: 18 nm or larger and 26 nm or smaller) and on the other hand, is capable of passing a protein, for example, a monoclonal antibody, having a size of 160 kD (approximately 8 nm) therethrough (see, for example, Patent Literature 3). For example, retrovirus (diameter: 80 nm or larger and 100 nm or smaller) can be removed using a membrane having a nominal pore size of 35 nm or larger.

In one embodiment, when the virus removal medium is a virus removal membrane, examples thereof include a virus removal membrane having a molecular weight cutoff of 100 kD or higher and 1000 kD or lower. In this context, the molecular weight cutoff (MWCO) of the membrane means a nominal molecular weight that enables 90% of a certain substance to pass through the membrane when the substance is passed through the membrane. A nominal molecular weight cutoff may be used as the molecular weight cutoff.

In one embodiment, the purified biological material-containing solution is not particularly limited as long as the solution has been harvested by contacting the virus removal medium with the biological material-containing solution before contact with the virus removal medium. The term "purified" can mean "contacted with the virus removal medium and harvested". The conditions of the biological material-containing solution described above can be appropriately applied to conditions of the purified biological material-containing solution.

In one embodiment, all viral capsids in a virus can be quantified by appropriately selecting the viral capsid-containing liquid used depending on the type of a viral quantification method or appropriately selecting a quantification method depending on the type of the virus used in evaluating the viral clearance capability of the virus removal medium.

In the case of adopting, for example, quantitative PCR as a quantification method for detecting a virus, a viral capsid-containing liquid from which empty viral particles have been removed is selected. The number of particles of the "virus having a nucleic acid" can be quantified by quantitative PCR, whereas the number of particles of the empty viral particles cannot be quantified by quantitative PCR. Since a virus usually incorporates one molecule of a nucleic acid per particle in a capsid, the number of nucleic acid molecules incorporated in the "virus having a nucleic acid" is equal to the number of the viral particles. In this respect, copies/mL can be used as a unit that indicates the number of nucleic acid molecules (copy number) incorporated in viral capsids.

In the case of adopting an infectivity measurement method as a viral quantification method, the ratio of the number of total viral capsid particles to infectivity in the viral capsid-containing liquid to be added to the solution to be purified is measured in advance. The number of total viral capsid particles in the solution to be purified before and after purification can be calculated with reference to the ratio (number of total viral capsid particles/infectivity) described above from infectivity measured in the solution to be purified before and after purification. In this case, empty viral particles do not have to be removed from the viral capsid-containing liquid.

The infectivity is a unit that indicates the concentration of an infectious virus. The infectivity measurement method includes an endpoint method which determines a minimum infectious unit, and a local lesion count method which calculates a local lesion formed by a virus. The endpoint method is generally a $TCID_{50}$ (tissue culture infectious dose 50) method, which involves serially diluting a virus, inoculating a given number or more of cultured cells with the dilutions, culturing the cells for a given period, and determining positivity/negativity of infection to determine a dilution ratio at which 50% of the cells are positive to infection. The local lesion count method is generally a plaque method, which involves inoculating a virus onto monolayer culture of host cells for virus adsorption, then overlaying thereon a culture medium containing agar which is then hardened, and measuring a plaque formed depending on the number of the inoculated virus. The unit of the infectivity can be $TCID_{50}$ when the $TCID_{50}$ method is used, and pfu can be used when the plaque method is used. The term "pfu" is an abbreviation of plaque forming unit. The units $TCID_{50}$/mL and pfu/mL indicate infectivity per mL.

The method for evaluating viral clearance capability is not limited to a specific method, and a logarithmic reduction value (LRV) calculation method or a viral capture capacity measurement method can be used.

In the LRV calculation method, a viral concentration is compared between before and after purification with the virus removal medium, and a logarithmic reduction value thereof is calculated. LRV can be calculated as follows.

LRV = Logarithmic value of the viral concentration before filtration - Logarithmic value of the viral concentration after filtration

The logarithmic value of the viral concentration before filtration is given by $\log_{10}$ [number of viral particles before filtration/mL]. The logarithmic value of the viral concentration after filtration is given by $\log_{10}$ [number of viral particles after filtration/mL].

In the viral capture capacity measurement method, a solution that has been contacted with the virus removal medium or has passed through the medium is separated as a plurality of fractions, and the viral nucleic acid concentration of each fraction is quantified. In this respect, a fraction in which the viral nucleic acid has been detected for the first time after contact with the virus removal medium or passage is selected. The total amount of virus loaded to the virus removal medium until the fraction is separated is regarded as the viral capture capacity of the virus removal medium.

(a) Step of adding viral capsid-containing liquid to solution to be purified

In one embodiment, the amount of a virus added (spiked) is not particularly limited as long as the amount does not cause abnormalities such as clogging in the virus removal medium due to the added viral capsid-containing liquid or impurities contained in the viral capsid-containing liquid when the virus removal medium is contacted with a biological material-containing solution supplemented with the viral capsid-containing liquid.

The amount of the viral capsid-containing liquid added, i.e., the rate of virus spiking, can be calculated as follows.

```
Rate of virus spiking (%) = (Volume of the added

viral capsid-containing liquid / Volume of the biological

material-containing solution to which the viral capsid-containing liquid is added) × 100
```

The rate of virus spiking is not particularly limited as long as the rate does not cause clogging in the virus removal medium after addition of the viral capsid-containing liquid. The rate of virus spiking for the biological material-containing solution may be, for example, 10% or less in terms of the upper limit and more than 0% in terms of the lower limit. The rate of virus spiking is preferably 1.0% or less from the viewpoint of preventing clogging in the virus removal medium with components other than viruses.

In the case of a biological material-containing solution aimed at commercial production, a biological material-containing solution produced under conditions scaled down at a proper ratio of production conditions for commercial production is preferred.

The temperature of the biological material-containing solution at which the viral capsid-containing liquid is added to the biological material-containing solution is not particularly limited as long as the biological material contained in the biological material-containing solution exists stably at the temperature. The temperature may be, for example, 50°C or lower, 37°C or lower, and 25°C or lower, in terms of the upper limit. The temperature may be, for example, higher than 0°C, 4°C or higher, and 8°C or higher, in terms of the lower limit. In the case of a biological material-containing solution aimed at commercial production, a production temperature for commercial production is preferred.

(b) Step of contacting virus removal medium with biological material-containing solution to harvest purified biological material-containing solution

In one embodiment, the contact of the virus removal medium with the biological material-containing solution is not particularly limited as long as the virus removal medium can be contacted with the biological material-containing solution. Examples thereof include the passage of the biological material-containing solution through the virus removal medium, and the dip of the virus removal medium in the biological material-containing solution. It is preferred to pass the biological material-containing solution through the virus removal medium from the viewpoint of relatively easily controlling the purification of the protein of interest.

In one embodiment, as passing the biological material-containing solution through the virus removal medium, for example, the biological material-containing solution is passed through the virus removal medium using a syringe or a pump. In the method for passing the biological material-containing solution through the virus removal medium, the biological material-containing solution injected to a certain portion of the virus removal medium may pass through the virus removal medium so that the biological material-containing solution can be harvested from another portion of the virus removal medium. Before or after passage of the biological material-containing solution through the virus removal medium, a buffer solution may be passed through the virus removal medium aside from the biological material-containing solution. When the biological material-containing solution is harvested, the whole biological material-containing solution that passed through the virus removal medium may be harvested (pool harvest), or fractions each having a given volume may be obtained (fractionation). In the case of fractionation, fractions containing a purified biological material can be collected and combined to harvest the purified biological material.

The flow rate at which the biological material-containing solution passes through the virus removal medium is not particularly limited. When the virus removal medium has the form of beads, the flow rate may be, for example, 0.1 mL/min or more per mL of the virus removal medium in terms of the lower limit. In another embodiment, the value is 0.5 mL/min or more. In an alternative embodiment, the value is 1.0 mL/min or more. In a further alternative embodiment, the value is 5 mL/min or more. When the virus removal medium is a medium having the form of a membrane, the flow rate may be, for example, more than 0 L/m$^2$/hour in terms of the lower limit. In another embodiment, the lower limit is 10 L/m$^2$/hour. In an alternative embodiment, the lower limit is 40 L/m$^2$/hour.

In one embodiment, as dipping the virus removal medium in the biological material-containing solution, for example, the virus removal medium is fed into the biological material-containing solution. In this respect, the biological material-containing solution to which the virus removal medium has been fed may be shaken with a shaker or the like or stirred with a stirring bar or the like, or the solution may be left standing. The virus removal medium thus dipped can be removed from the biological material-containing solution to harvest a purified protein. Examples of the method for removing the virus removal medium include centrifugation and filtration.

In one embodiment, harvesting the purified biological material-containing solution is not particularly limited as long as the purified biological material can be obtained. The purified biological material can be harvested in the state of a solution where the biological material is dissolved. The harvest can be carried out by appropriately selecting a method such as pool harvest or fractionation.

In one embodiment, the virus removal medium described above can be used as the virus removal medium.

(c) Step of quantifying total viral capsid in biological material-containing solution before purification and total viral capsid in purified biological material-containing solution

In one embodiment, in order to more accurately evaluate the viral clearance capability of a virus removal medium, a viral quantification method capable of measuring a total viral capsid present in the biological material-containing solution can be selected, or a quantification method can be appropriately selected depending on the type of the virus used in the viral clearance capability evaluation. Examples of the viral quantification method include quantitative PCR, fluorescence flow cytometry, a $TCID_{50}$ method, and a plaque method.

In the case of adopting, for example, a method for detecting a viral nucleic acid, such as quantitative PCR, as a viral quantification method, a viral capsid-containing liquid from which empty viral particles have been removed can be selected. In the case of adopting a method for measuring viral infectivity, such as an infectivity measurement method, as a viral quantification method, the ratio of the number of total viral capsid particles to infectivity in the viral capsid-containing liquid to be added to the biological material-containing solution (number of total viral capsid particles/infectivity) is measured in advance, and the number of total particles in the biological material-containing solution can be calculated with reference to the ratio (number of total viral capsid particles/infectivity) described above from infectivity measured in the biological material-containing solution.

(c1) Step of quantifying nucleic acid of virus in biological material-containing solution before purification and nucleic acid of virus in purified biological material-containing solution

In this case, a viral capsid-containing liquid from which empty viral particles have been removed (e.g., a FC viral capsid-containing liquid) can be used as the viral capsid-containing liquid to be added in the step (a).

A known method can be used as a method for quantifying a nucleic acid. Examples thereof include quantitative PCR and fluorescence flow cytometry. Quantitative PCR is more preferred, and quantitative real-time PCR is further preferred. In the quantitative PCR, for example, a product LightCycler 96 (manufactured by F. Hoffmann-La Roche, Ltd.) can be used. In the fluorescence flow cytometry, for example, a product Gallios (manufactured by Beckman Coulter Inc.) can be used.

In the case of using quantitative PCR, a known method can be used as a method for extracting a nucleic acid from a virus. Examples thereof include methods involving use of various nucleic acid extraction kits, and a phenol-chloroform extraction method. A method involving use of a nucleic acid extraction kit is preferred, and a method involving use of High Pure Viral Nucleic Acid Extraction Kit (manufactured by F. Hoffmann-La Roche, Ltd.) is more preferred.

The amount of a sample subjected to nucleic acid extraction is not particularly limited. The concentration of the nucleic acid contained in the sample subjected to nucleic acid extraction can be calculated through the use of the amount of the sample supplied to nucleic acid extraction, and the number of actually detected nucleic acid molecules.

Prior to nucleic acid extraction, it is preferred to remove in advance a free nucleic acid contained in the harvested biological material-containing solution. As a result, the viral clearance capability of the virus removal medium can be more accurately evaluated. A method including degrading the free nucleic acid using nuclease can be used as a method for removing the free nucleic acid in advance. The type of the nuclease is not particularly limited, and nuclease suitable for the properties of the biological material-containing solution can be selected and used. For example, nuclease such as DNaseI (Sigma-Aldrich Co. LLC) or Benzonase (Merck KGaA) can be used.

An absolute quantification method involving use of a nucleic acid having a known concentration as a standard can be used as a method for quantifying a nucleic acid by quantitative PCR. In this respect, a nucleic acid that can be amplified by PCR using a primer or a probe for use in the measurement of a target sample can be used as the nucleic acid having a known concentration. A nucleic acid having a nucleotide sequence completely identical to the nucleotide sequence of a target sample is preferred, and a nucleic acid having a nucleotide sequence partially identical to the nucleotide sequence of a target sample is more preferred.

The viral clearance capability of the virus removal medium can be evaluated by quantifying the viral nucleic acid contained in the biological material-containing solution spiked with the viral capsid-containing liquid from which empty viral particles have been removed, and the viral nucleic acid contained in the purified biological material-containing solution. As described above, quantitative PCR or fluorescence flow cytometry can be used as a method for quantifying a nucleic acid. More preferably, quantitative PCR, further preferably quantitative real-time PCR, can be used. The removal of empty viral particles having no nucleic acid can prevent the empty viral particles which are not detected by the method for quantifying a nucleic acid from influencing the viral clearance capability evaluation of the virus removal medium.

(c2) Step of quantifying infectivity of virus in biological material-containing solution before purification and infectivity of virus in purified biological material-containing solution, and calculating number of total viral capsid particles in biological material-containing solution before purification and number of total viral capsid particles in purified biological material-containing solution from ratio T of number of total viral capsid particles to infectivity (number of total viral capsid particles/infectivity) in viral capsid-containing liquid to be added to biological material-containing solution

Ratio T of the number of total viral capsid particles to infectivity (= number of total viral capsid particles/infectivity) in the viral capsid-containing liquid to be added to the biological material-containing solution is calculated, for example, before or after quantification of infectivity. In the case of the step (c2), the viral capsid-containing liquid to be added to the biological material-containing solution in the step (a) may contain a non-infectious virus and/or empty viral particles in addition to an infectious virus. Thus, empty viral particles do not have to be removed from the viral capsid-containing liquid.

Any known infectivity measurement method can be selected as a method for measuring the "infectivity". For example, a $TCID_{50}$ method or a plaque method can be used. The method for measuring the infectivity of a virus in the biological material-containing solution before purification, the method for measuring the infectivity of a virus in the purified biological material-containing solution, and the method for measuring the infectivity of a virus in the viral capsid-containing liquid to be added to the biological material-containing solution are preferably the same with each other.

The ratio T is defined as a value obtained by dividing the "number of total viral capsid particles" by the "infectivity" in the viral capsid-containing liquid to be added to the biological material-containing solution in the step (a). Specifically, this ratio means the number of total viral capsid particles per infectivity in the viral capsid-containing liquid to be added to the biological material-containing solution. In short, use of the ratio T enables the number of total viral capsid particles in the biological material-containing solution to be calculated from the infectivity of a virus in the biological material-containing solution.

The ratio T may be calculated at any timing before or after addition of the viral capsid-containing liquid to the biological material-containing solution in the step (a). However, if the execution of the step (a) makes it impossible to obtain the same viral capsid-containing liquid as that added in the step (a) (e.g., the viral capsid-containing liquid is consumed), the ratio T needs to be calculated in advance before the step (a) .

Any method can be selected as a method for measuring the "number of total viral capsid particles" in the definition of the ratio T as long as the method is capable of measuring the concentration (number/mL) of a viral capsid present as particles. For example, a method including measuring the capsid protein weight of viral capsid particles and calculating the number of particles, or a measurement of the particle concentration under a transmission electron microscope can be used. Examples of the method for measuring the capsid protein weight of viral capsid particles include Western blot and ELISA (enzyme linked immunosorbent assay).

In general, the weight of a capsid protein constituting one viral capsid particle can be determined by calculation. When the virus used is, for example, MVM (minute virus of mice), it is known that 50 particles in total of VP2 and VP3 proteins which are capsid proteins are contained in one particle of the virus (see, for example, Non Patent Literatures 10 and 11). The molecular weight of VP2 is approximately 64300 g/mol, and the molecular weight of VP3 is 61400 g/mol (see, for example, Non Patent Literature 11). The content ratio of these molecules in MVM particles generally has a much larger proportion of VP2. Therefore, the molecular weight 64300 g/mol of VP2 can be used as a representative molecular weight between these proteins. In consideration of an Avogadro's constant of 6.0E23/mol, the total content of VP2 and VP3 per one MVM particle can be calculated as (64300 g/mol × 50) / 6.0E23/mol = 5.4E-18 g. Specifically, in the step (a), the total weight of VP2 and VP3 contained in the viral capsid-containing liquid to be added is quantified, and the value can be divided by 5.4E-18 to calculate the number and concentration of MVM particles.

Before quantification of the capsid protein weight, it is preferred to remove in advance capsid proteins that do not form particle structures among capsid proteins contained in the viral capsid-containing liquid. The capsid protein that does not form particle structure has a small particle size as compared with viral capsid particles, and can therefore be removed by a separation method that eliminates capsid proteins while maintaining viral capsid particles. Examples of this method include methods such as dialysis, desalting, and filtration. Dialysis is preferred. Dialysis with Float-A-Lyzer G2, CE, 1 mL, 1000 KD (Funakoshi Co., Ltd.) is more preferred.

In order to calculate the ratio T given by {number of total viral capsid particles (number)/infectivity ($TCID_{50}$ or Pfu)}, the real value of the "number of total viral capsid particles" per unit volume such as 1 mL can be divided by the real value of the "infectivity" per unit volume such as 1 mL selected above.

Next, the infectivity of a virus in the biological material-containing solution before purification and the infectivity of a virus in the purified biological material-containing solution are quantified. In this respect, the same method as that used for calculating the ratio T can be selected as a method for quantifying the viral infectivity.

Further, the viral infectivity in the biological material-containing solution before purification and the viral infectivity in the purified biological material-containing solution can be multiplied by the ratio T, to thereby convert the viral infectivity in the biological material-containing solution before purification and the viral infectivity in the purified biological material-containing solution to the respective number of total viral capsid particles.

Examples

Hereinafter, the present invention will be described in more detail with reference to Examples and Comparative Examples. Examples given below are typical examples, and the present invention is not limited by Examples given below.

1. Production of viral concentrate - (1)

Simian virus 40-transformed human fibroblasts were used as host cells for minute virus of mice (MVM), and subcultured in DMEM supplemented with 5% fetal bovine serum at 37°C in a 5% $CO_2$ environment using a flask for tissue culture having a bottom area of 75 cm$^2$ and a capacity of 15 mL (hereinafter, simply referred to as a "flask"). The simian virus 40-transformed human fibroblasts were detached from the flask and implanted at a density of 1.5 × 10^6 cells/15 mL/flask to DMEM medium containing 2.5% FBS in a fresh flask so that the cells were infected with MVM at MOI = 0.01. Four days later, the medium was replaced with a serum-free medium, and the cells were further cultured for three days. Three days after infection, a culture supernatant containing MVM was harvested. After centrifugation at 3000 rpm for 20 minutes, supernatant fractions were harvested, and this supernatant was filtered through a 0.45 $\mu$m filter (manufactured by Nalgene/Nalge Nunc International Corp.) to obtain a viral culture solution. This operation was carried out as to 72 flasks to obtain 720 mL in total of viral culture solutions.

The resulting viral culture solution was ultracentrifuged at 29400 rpm for two hours in an ultracentrifuge (Optima L-90K manufactured by Beckman Coulter Inc.) equipped with Type 45 Ti rotor to precipitate minute virus of mice. After removal of a supernatant, pellets containing minute virus of mice were resuspended by the addition of 30 mL of a TE buffer solution (50 mmol/L Tris-HCl pH 8.0 / 1 mmol/L EDTA) to obtain 30 mL of a TE buffer solution containing concentrated MVM. This operation was repeated again to obtain 60 mL in total of TE buffer solutions containing concentrated MVM.

Next, the resulting TE buffer solution containing concentrated MVM was suspended by the addition of PEG6000 and NaCl at a final concentration of 10 w/v% and a final concentration of 500 mmol/L, respectively, and left standing overnight at 4°C. This solution was centrifuged at 7800 rpm for 30 minutes in an ultracentrifuge (Optima L-90K manufactured by Beckman Coulter Inc.) equipped with SW28 rotor to precipitate minute virus of mice. After removal of a supernatant, pellets containing minute virus of mice were resuspended by the addition of 1.2 mL of PBS + KM (1 × PBS / 2.5 mmol/L KCl / 1 mmol/L $MgCl_2$) to obtain a solution containing concentrated MVM (hereinafter, referred to as "MVM-PEG").

2. Production of FC viral capsid-containing liquid

MVM-PEG was purified by iodixanol density gradient ultracentrifugation. iodixanol/PBS + KM was overlaid in the order of 55%-2 mL, 45%-2 mL, 40%-2 mL, 35%-2 mL, 25%-2 mL, and 15%-1 mL from the bottom in a UC tube from Beckman Coulter Inc. (13.2 mL, 344059), and 1 mL of MVM-PEG was overlaid at the uppermost part. This sample was ultracentrifuged at 35000 rpm at 18°C for 18 hours in an ultracentrifuge (Optima L-90K manufactured by Beckman Coulter Inc.) equipped with SW41 rotor. Then, 1 mL each of 12 fractions was harvested from the upper part of the tube. A density, $TCID_{50}$/mL, the number of MVM nucleic acid copies, and chicken hemagglutination (HA) activity were measured for a total of 12 fractions thus harvested. In the measurement of the number of MVM nucleic acid copies, a free nucleic acid contained in the sample was removed by nuclease treatment, and the number of molecules of the MVM nucleic acid obtained by the destruction of capsids was then measured by quantitative real-time PCR. The number of molecules of the viral nucleic acid was quantified by using a nucleic acid having a known concentration as a standard. The MVM standard used was linear double-stranded DNA obtained by cloning 4775 nucleotides, except for a repeat sequence, of ATCC VR1346 strain into pT7Blue-2 T-vector (manufactured by Novagen, Inc., product number 69080), followed by the cleavage of the plasmid with restriction enzymes SalI-HF and EcoNI (manufactured by New England BioLabs Inc., product numbers R3138S and R0521S). The chicken hemagglutination activity is a method for detecting a specimen having hemagglutinin. The viral liquid was serially diluted two-fold, inoculated to preserved chicken blood (Nippon Bio-Test Laboratories Inc.) diluted to 0.13% with PBS, and left standing at 25°C for two hours. A limited dilution ratio at which the sample was positive to erythrocyte agglutination was determined to measure the number of hemagglutinin particles, i.e., a viral capsid particles, present in the sample.

Fraction 10 which peaked in all of the indexes $TCID_{50}$/mL, the number of MVM nucleic acid copies, and HA activity was harvested as a FC viral capsid-containing liquid, and fractions 5 and 6 in which only the HA activity appeared were harvested as empty viral particles-containing liquids.

Subsequently, the solvents of the fractions 5 and 6 or 10 were each changed from iodixanol/PBS + KM to PBS + KM using Zeba Spin Desalting Column 40K MWCO 10 mL (Thermo Fisher Scientific Inc.) to obtain 4.6 mL of a MVM EC viral capsid-containing liquid (MVM EC) and 2.1 mL of a MVM FC viral capsid-containing liquid (MVM FC).

Table 1 shows the logarithmic value of the number of viral nucleic acid molecules per mL (Log$_{10}$ [copies/mL]), and the limited dilution ratio at which the sample was positive to chicken hemagglutination as to each of the MVM FC and the MVM EC. As a result of cryo-electron microscopy, the ratio of empty viral particles contained in the MVM FC was less than 1.0%, and the ratio of a virus having a nucleic acid contained in the MVM EC was less than 0.5%.

[Table 1]

|  | Log$_{10}$[copies/mL] | HA-positive limited dilution ratio (fold) |
|---|---|---|
| MVM FC | 12.27 | 3200 |
| MVM EC | 9.83 | 3200 |

3. Pharmaceutical formulation of viral capsid-containing liquid that contains empty viral particle

The MVM FC and the MVM EC were mixed to prepare a viral capsid-containing liquid that contained empty viral particles at an arbitrary ratio (referred to as a "FE mixed liquid"). The mixing ratio between the MVM FC and the MVM EC was determined on the basis of the concentration of a total viral capsid contained in each viral capsid-containing liquid, which was calculated from the HA-positive limited dilution ratio. For example, since the MVM FC is a viral capsid-containing liquid that contains no empty particle, the total concentration of viral particles contained therein is equal to Log$_{10}$ [copies/mL]. In this respect, the total concentration of viral particles contained in the MVM FC is 12.27 Log$_{10}$ [particles/mL]. In this context, since the HA-positive limited dilution ratio is proportional to the concentration of a total viral capsid contained in the viral capsid-containing liquid, the concentration of a total viral capsid contained in the MVM EC can be calculated as 12.27 Log$_{10}$ [particles/mL] from the comparison of the HA-positive limited dilution ratio between the MVM FC and the MVM EC.

Table 2 shows the FE mixed liquid, the ratio between MVM FC and MVM EC mixed for pharmaceutical formulation thereof, and the logarithmic value of the number of viral nucleic acid molecules per mL (Log$_{10}$[copies/mL]).

[Table 2]

|  | Amount of MVM FC mixed (mL) | Amount of MVM EC mixed (mL) | Mixing ratio between MVM FC and MVM EC | Log$_{10}$ [copies/mL] |
|---|---|---|---|---|
| FE mixed liquid (1) | 0.20 | 1.00 | 1:5 | 11.53 |
| FE mixed liquid (2) | 0.10 | 1.00 | 1:10 | 11.21 |
| FE mixed liquid (3) | 0.02 | 2.00 | 1:100 | 10.29 |

4. Production of viral concentrate - (2)

A viral culture solution was obtained in the same manner as in 1. Production of viral concentrate - (1). This viral culture solution was ultracentrifuged at 29400 rpm for 2 hours in an ultracentrifuge (Optima L-90K manufactured by Beckman Coulter Inc.) equipped with Type 45 Ti rotor to precipitate minute virus of mice. After removal of a supernatant, pellets containing minute virus of mice were resuspended by the addition of 6 mL of a TNE buffer solution (50 mmol/L Tris-HCl pH 8.0 / 100 mmol/L NaCl / 1 mmol/L EDTA) to obtain 6 mL of a TNE buffer solution containing concentrated MVM (hereinafter, referred to as a "concentrated MVM culture solution (1)". This operation was repeated twice to further obtain two concentrated MVM culture solutions (concentrated MVM culture solution (2) and concentrated MVM culture solution (3)).

5. Calculation of ratio T of concentrated viral culture solution

1.7 μL of 4 × Laemmli sample buffer (Bio-Rad Laboratories, Inc.) was added to 5 μL of the concentrated MVM culture solution (concentrated MVM culture solution (1)) and to 5 μL of dilution series of 3.5 ng/μL recombinant MVM-VP2 standard (MVMVP21-C manufactured by Alpha Diagnostics Ltd.; N-terminally His6-tagged MVM-VP2), and incubated at 95°C for five minutes. These samples were separated by SDS-PAGE using 10% Mini-Protean TGX Precast Gels (Bio-Rad Laboratories, Inc.). The 10% Mini-Protean TGX Precast Gels after the separation were collected, and proteins were transferred to a membrane under conditions of 25 V constant voltage and 30 minutes using Transblot Turbo blotting system (Bio-Rad Laboratories, Inc.) and Transblot Turbo RTA nitrocellulose transfer kit (Bio-Rad Laboratories, Inc.). After the completion of transfer, the membrane was collected, dipped in 5% skimmed milk/phosphate buffered saline with Tween 20 (PBST, Takara Bio Inc.), and shaken at room temperature for 1 hour. This membrane was washed three times with PBST, dipped in 5000-fold diluted anti-MVM-VP2 antibody (MVMVP21-S manufactured by Alpha Diagnostics Ltd.)/PBST, and reacted overnight at 4°C. Then, the membrane was washed three times with PBST, dipped in 5000-fold diluted HRP-conjugated goat antirabbit IgG antibody (Thermo Fisher Scientific Inc.)/PBST, and reacted at room temperature for 1 hour. The membrane was washed three times with PBST, followed by the detection of MVM-VP2 and -VP3 signals, as shown in Figure 1, using SuperSignal WEST Femto maximum sensitivity substrate (Thermo Fisher Scientific Inc.) and ChemiDoc XRS Plus (Bio-Rad Laboratories, Inc.).

The MVM-VP2 and MVM-VP3 signals detected from the concentrated MVM culture solution and the MVM-VP2 standard samples were quantified using ImageJ (NIH). First, the signals from the recombinant VP2 standard having a known concentration were quantified to prepare a calibration curve of the amounts of VP2 (ng) against the signal values. Next, the signals from the concentrated MVM culture solution were quantified, and the masses of MVM-VP2 and -VP3 contained in the concentrated MVM culture solution were determined with reference to the calibration curve prepared from the signals of the recombinant VP2 standard. In consideration of 50 molecules in total of MVM-VP2 (64.3 kDa) and -VP3 contained per MVM1 particle, the MVM concentration in the concentrated MVM culture solution (logarithmic value of the number of MVM particles per unit volume of the concentrated MVM culture solution: $\mathrm{Log}_{10}$[particles/mL]) was calculated from the total amount of the MVM-VP2 and - VP3 proteins in the concentrated MVM culture solution. The calculation results are shown in Table 3.

The MVM concentration was further calculated from the SDS-PAGE as to the two additional concentrated MVM culture solutions (concentrated MVM culture solution (2) and concentrated MVM culture solution (3)). The calculation results are shown in Table 3.

[Table 3]

| | Total amount of MVM-VP2 and -VP3 proteins (ng/5 $\mu$L) | MVM concentration ($\mathrm{Log}_{10}$ [particles/mL]) |
|---|---|---|
| Concentrated MVM culture solution (1) | 11.16 | 11.55 |
| Concentrated MVM culture solution (2) | 7.28 | 11.37 |
| Concentrated MVM culture solution (3) | 1.30 | 10.55 |

Infectivity per mL of the concentrated MVM culture solution ($\mathrm{Log}_{10}$[$\mathrm{TCID}_{50}$/mL]) was separately measured, and the common logarithm of ratio T of the number of viral particles to the infectivity of the concentrated MVM culture solution, i.e., $\mathrm{Log}_{10}$[(particles)/($\mathrm{TCID}_{50}$)] was calculated. The calculation results are shown in Table 4.

[Table 4]

| | $\mathrm{Log}_{10}$[$\mathrm{TCID}_{50}$/mL] | $\mathrm{Log}_{10}$[particles/mL] | $\mathrm{Log}_{10}$[(number of viral particles/ infectivity)] |
|---|---|---|---|
| Concentrated MVM culture solution (1) | 9.00 | 11.55 | 2.55 |
| Concentrated MVM culture solution (2) | 9.00 | 11.37 | 2.37 |
| Concentrated MVM culture solution (3) | 8.08 | 10.55 | 2.47 |

[Example 1]

1 mL of the FE mixed liquid (1) was purified by ultracentrifugation in accordance with the method described in "2. Production of FC viral capsid-containing liquid" to obtain a viral capsid-containing liquid that contained no empty virus (hereinafter, referred to as a "FE mixed liquid (1)-E"). Likewise, viral capsid-containing liquids that contained no empty virus (hereinafter, referred to as a "FE mixed liquid (2)-E" and a "FE mixed liquid (3)-E") were obtained from 1 mL of the FE mixed liquid (2) and 1 mL of the FE mixed liquid (3), respectively. As a result of cryo-electron microscopy, the ratios of empty viral particles contained in the FE mixed liquid (1)-E and the FE mixed liquid (2)-E were both less than 1.0%.

The FE mixed liquid (1)-E was added to a biological material-containing solution (pH 4.5) containing 10 mg/mL (final concentration) human globulin and 0.1 mol/L NaCl (a total of 100 mL), and this solution was filtered through Planova 20N (manufactured by Asahi Kasei Medical Co., Ltd., membrane area: 0.001 m2). The number of MVM nucleic acid copies per mL of the biological material-containing solution supplemented with the FE mixed liquid (1)-E was 8.87 $Log_{10}$[copies/mL]. The filtration conditions involved 78.4 kPa and 25°C.

A fraction size was determined such that the amount of virus load per membrane area given by $Log_{10}$[copies/m2] has an interval of approximately 0.2. A total of 12 fractions were harvested from the filtrate after passage through the virus removal membrane. A 230 $\mu$L aliquot was separated from each of the liquid before passage through the virus removal membrane, and the virus removal membrane filtrate thus fractionated, and 25 $\mu$L of 10 $\times$ reaction buffer (500 mmol/L Tris-HCl pH 8.0, 150 mmol/L $CaCl_2$, 50 mmol/L $MgCl_2$) and 40 units/$\mu$L (final concentration) of Benzonase (F. Hoffmann-La Roche, Ltd.) were added thereto and left standing at room temperature for 1 hour. After this reaction, 10 mmol/L (final concentration) EDTA was added thereto and left standing at room temperature for 10 minutes. A nucleic acid was extracted from this sample using High Pure Viral Nucleic Acid Kit (F. Hoffmann-La Roche, Ltd.). The concentration of the extracted nucleic acid was measured by quantitative real-time PCR to quantify a viral nucleic acid, i.e., the number of viral particles, in the liquid before passage through the virus removal membrane and the fractions of the virus removal membrane filtrate.

A fraction was selected such that a virus was detected for the first time in the filtrate fraction and a viral nucleic acid was continuously detected in subsequent fractions. The viral nucleic acid concentration of the liquid before passage through the virus removal membrane was multiplied by the total filtration volume until the fraction was harvested to calculate a limited amount of virus load without virus leakage. This limited amount of virus load was regarded as the virus retention capacity of the virus removal membrane. The virus retention capacity indicates the limit value of the number of viral particles removable by the virus removal membrane and as such, can be used as an indicator for the viral clearance capability of the virus removal membrane.

[Table 5]

| Fraction No. [A] | Total filtration volume (mL) [B] | Total amount of virus load per unit membrane area (Log [copies/m$^2$]) [C] | Virus detection [D] |
|---|---|---|---|
| 1 | 1.71 | 12.10 | Negative |
| 2 | 3.01 | 12.35 | Negative |
| 3 | 4.71 | 12.54 | Negative |
| 4 | 7.58 | 12.75 | Negative |
| 5 | 11.73 | 12.94 | Negative |
| 6 | 17.39 | 13.11 | Positive |
| 7 | 26.36 | 13.29 | Positive |
| 8 | 41.05 | 13.48 | Positive |
| 9 | 65.73 | 13.69 | Positive |
| 10 | 100.00 | 13.87 | Positive |

The filtration results are shown in Table 5. In Table 5, the number of viral nucleic acid copies per mL of the liquid before passage through the virus removal membrane (8.87 = antilogarithm of $Log_{10}$[copies/mL]) was multiplied by the column [B] of the total filtration volume and further divided by the membrane area 0.001 m$^2$ to calculate the column [C] of the total amount of virus load per unit area upon separation of the fraction. These fractions were subjected to virus detection (column [D]) by quantitative real-time PCR. Fraction 5 immediately before a viral nucleic acid was detected for the first time in a filtrate fraction and continuously detected in subsequent fractions was selected. The total amount 12.94 of virus load per unit membrane area given by $Log_{10}$[copies/m$^2$] upon collection of the fraction 5 was regarded as the virus retention capacity of the virus removal membrane.

The FE mixed liquid (2)-E and the FE mixed liquid (3)-E were further filtered in the same manner as in the FE mixed liquid (1)-E, and the virus retention capacity of the virus removal membrane was determined. The number of MVM nucleic acid copies per mL of the biological material-containing solution supplemented with the FE mixed liquid (2)-E was 8.68 $Log_{10}$[copies/mL], and the number of MVM nucleic acid copies per mL of the biological material-containing solution supplemented with the FE mixed liquid (3)-E was 7.91 $Log_{10}$[copies/mL]. The fraction immediately before a viral nucleic acid was detected for the first time in a filtrate fraction and continuously detected in subsequent fractions was fraction 6 for the FE mixed liquid (2)-E and fraction 10 for the FE mixed liquid (3)-E. The total filtration volume until the fraction was 17.36 mL for the FE mixed liquid (2)-E and 105.73 mL for the FE mixed liquid (3)-E. Table 6 shows results about the virus retention capacity of the virus removal membrane when each mixed liquid was used.

[Table 6]

|  | Virus retention capacity of Planova 20N ($Log$[copies/m$^2$]) |
|---|---|
| FE mixed liquid (1)-E | 12.94 |
| FE mixed liquid (2)-E | 12.92 |
| FE mixed liquid (3)-E | 12.94 |

As is evident from these results, the virus retention capacity of a virus removal membrane can be accurately measured by removing empty particles and adopting quantitative PCR measurement, even if viral capsid-containing liquids differing in the percent content of empty particles before removal (FE mixed liquids (1) to (3)) are used. In this context, the total amounts of virus load per unit membrane area ($Log$[copies/m$^2$]) until the fraction for which a viral nucleic acid was detected for the first time in the filtrate fraction and consequently detected in subsequent fractions, and until the fraction immediately therebefore were

12.94 and 13.11, respectively, for the FE mixed liquid (1)-E,
12.92 and 13.10, respectively, for the FE mixed liquid (2)-E, and
12.94 and 13.13, respectively, for the FE mixed liquid (3)-E.

The total amount of virus load per unit membrane area given by $Log$[copies/m$^2$] was increased by 0.17 to 0.19 per fraction. Hence, a total amount of virus load at which virus leakage starts cannot be identified within the range of 0.19 from the separation of the fraction immediately before a viral nucleic acid is detected for the first time in a filtrate fraction and continuously detected in subsequent fractions. Specifically, in this Example, a difference of less than 0.19 is accepted for the virus retention capacity given by $Log$[copies/m$^2$].

By contrast, in this Example, the virus retention capacity for the MVM FC viral capsid-containing liquid given by $Log$[copies/m$^2$] was 12.94 as the maximum value and 12.92 as the minimum value, the difference between which was 0.02. This is smaller than 0.19. Therefore, in this Example, the difference in virus retention capacity among the FE mixed liquid (1)-E, the FE mixed liquid (2)-E, and the FE mixed liquid (3)-E is accepted as an error. Specifically, it is evident that the viral clearance capability of a virus removal membrane can be accurately evaluated by the present method even for a viral capsid-containing liquid containing any concentration of empty particles.

[Example 2]

The concentrated MVM culture solution (1) containing FC virus and empty viral particles was added to a biological material-containing solution (pH 4.5) containing 10 mg/mL (final concentration) human globulin and 0.1 mol/L NaCl (a total of 250 mL) such that infectivity per mL given by $Log_{10}$[TCID$_{50}$/mL] was 4.208. The biological material-containing solution supplemented with the viral capsid-containing liquid was filtered through a virus removal membrane (cellulose membrane produced in accordance with the method described in Japanese Patent No. 6220447; average water permeation pore size: 20 nm, membrane area: 0.001 m$^2$). The filtration conditions involved 78.4 kPa and 25°C.

A fraction size was determined such that the amount of virus load per membrane area given by $Log_{10}$[TCID$_{50}$/m$^2$] became an interval of 0.2. A total of 12 fractions were harvested from the filtrate after passage through the virus removal membrane. Infectivity per mL of each of the liquid before passage through the virus removal membrane, and the virus removal membrane filtrate thus fractionated ($Log_{10}$[TCID$_{50}$/mL]) was measured.

A fraction immediately before an infectious virus was detected for the first time in a filtrate fraction and continuously detected in subsequent fractions was selected. The MVM infectivity per mL of the liquid before passage through the virus removal membrane was multiplied by the total filtration volume until the fraction was harvested to calculate a limited amount of virus load without virus leakage. This limited amount of virus load was regarded as the virus infectivity retention capacity of the virus removal membrane.

Table 7 shows the results of filtering the concentrated MVM culture solution (1). In Table 7, the viral infectivity per mL of the liquid before passage through the virus removal membrane (4.208 = antilogarithm of $Log_{10}[TCID_{50}/mL]$) was multiplied by the column [B] of the total filtration volume and further divided by the membrane area 0.001 $m^2$ to calculate the column [D] of the total amount of virus load per unit area upon separation of the fraction. These fractions were subjected to virus detection (column [E]) by the $TCID_{50}$ method. Fraction 6 immediately before an infectious virus was detected for the first time in a filtrate fraction and continuously detected in subsequent fractions was selected. The total amount 8.61 of virus load per unit membrane area given by $Log_{10}[TCID_{50}/m^2]$ upon collection of the fraction 6 was regarded as the virus infectivity retention capacity of the virus removal membrane.

[Table 7]

| Fraction No. [A] | Total filtration volume (mL) [B] | Fraction size (mL) [C] | Total amount of virus load per unit membrane area ($Log_{10}[TCID_{50}/m^2]$) [D] | Virus detection using $TCID_{50}$ method in filtrate fraction [E] |
|---|---|---|---|---|
| 1 | 2.52 | 2.52 | 7.61 | Negative |
| 2 | 4.28 | 1.76 | 7.84 | Negative |
| 3 | 6.85 | 2.57 | 8.04 | Negative |
| 4 | 10.38 | 3.53 | 8.22 | Negative |
| 5 | 16.16 | 5.78 | 8.42 | Negative |
| 6 | 25.13 | 8.97 | 8.61 | Negative |
| 7 | 38.64 | 13.51 | 8.80 | Positive |
| 8 | 62.31 | 23.67 | 9.00 | Positive |
| 9 | 97.70 | 35.39 | 9.20 | Positive |
| 10 | 150.27 | 52.57 | 9.38 | Positive |
| 11 | 219.83 | 69.56 | 9.55 | Positive |

Next, the virus infectivity retention capacity of the virus removal membrane was converted to a virus retention capacity. Since the virus retention capacity indicates the limit value of the "number" of viruses removable by the virus removal membrane, the virus infectivity retention capacity can be converted to the virus retention capacity by adding the logarithmic value of the ratio T (number of total viral capsid particles/infectivity) to the virus infectivity retention capacity given by $Log_{10}[TCID_{50}/m^2]$. The logarithmic value 2.55 of the ratio T of the number of total viral capsid particles to the infectivity of the concentrated MVM culture solution determined beforehand was added to the virus infectivity retention capacity 8.61 given by $Log_{10}[TCID_{50}/m^2]$, and the resulting value 11.16 was regarded as the virus retention capacity given by $Log_{10}[particles/m^2]$.

The concentrated MVM culture solution (2) and the concentrated MVM culture solution (3) shown in Table 4 were further filtered in the same manner as in the concentrated MVM culture solution (1), and the virus retention capacity of the virus removal membrane was determined. The fraction immediately before an infectious virus was detected for the first time in a filtrate fraction and continuously detected in subsequent fractions was fraction 8 for the concentrated MVM culture solution (2) and fraction 8 for the concentrated MVM culture solution (3). The total filtration volume until the fraction was 38.99 mL for the concentrated MVM culture solution (2) and 39.55 mL for the concentrated MVM culture solution (3). Table 8 shows results about the virus retention capacity of the virus removal membrane when each liquid was used.

[Table 8]

| | Virus retention capacity of virus removal membrane ($Log[particles/m^2]$) |
|---|---|
| Concentrated MVM culture solution (1) | 11.16 |
| Concentrated MVM culture solution (2) | 10.86 |
| Concentrated MVM culture solution (3) | 11.27 |

In this Example, an infectious virus was detected by the $TCID_{50}$ method, and the virus infectivity retention capacity was then converted to the virus retention capacity using the ratio T. In this case, an error in virus retention capacity for the concentrated MVM culture solutions (1) to (3) was brought about by the error of the $TCID_{50}$ method. In the $TCID_{50}$ method, an experimental error of $\pm 0.25$ generally occurs in a measurement value given by $Log_{10}[TCID_{50}/mL]$. Thus, in the case of comparing two different measurement values, a difference within 0.5 is accepted as an experimental error. As shown in Table 8, in this Example, the virus retention capacity was 11.27 as the maximum value and 10.86 as the minimum value, the difference between which was 0.41. This difference is smaller than 0.5 accepted as an error. Therefore, in this Example, it can be confirmed that the virus retention capacity for the concentrated MVM culture solutions (1) to (3) can be accurately measured without an error.

As is evident from these results, according to this method, the virus retention capacity of a virus removal membrane can be accurately measured as a value based on the number of particles even if viral capsid-containing liquids variously differing in the number of total viral capsid particles relative to infectivity among pharmaceutical formulation lots are used in filtration.

As is also evident from these results, the virus retention capacity of a virus removal membrane can be accurately measured without an error even for viral capsid-containing liquids differing in pharmaceutical formulation lot (MVM (1) to (3)), by converting quantified infectivity to a viral concentration using the ratio T of the viral capsid-containing liquid used, even if the viral clearance of the virus removal membrane is evaluated by adopting the $TCID_{50}$ method which can detect infectivity by the addition of a virus to a human globulin-containing solution. Specifically, it is evident that the viral clearance capability of a virus removal membrane can be accurately evaluated by the present method.

[Comparative Example 1]

The FE mixed liquid (1) shown in Table 2 was added to a biological material-containing solution (pH 4.5) containing 10 mg/mL (final concentration) human globulin and 0.1 mol/L NaCl (a total of 200 mL), and this solution was filtered through Planova 20N (manufactured by Asahi Kasei Medical Co., Ltd., membrane area: 0.001 $m^2$). The number of MVM nucleic acid copies per mL of the biological material-containing solution supplemented with the FE mixed liquid (1) was 7.76 $Log_{10}[copies/mL]$. The filtration conditions involved 78.4 kPa and 25°C. The collection of fractions and the quantification of a viral nucleic acid were carried out in the same manner as the method described in Example 1 except that the FE mixed liquid (1) was used. The filtration results are shown in Table 9.

[Table 9]

| Fraction No. [A] | Total filtration volume (mL) [B] | Total amount of virus load per unit membrane area (Log [copies/m$^2$]) [C] | Virus detection [D] |
|---|---|---|---|
| 1 | 1.59 | 10.96 | Negative |
| 2 | 2.9 | 11.22 | Negative |
| 3 | 4.54 | 11.42 | Negative |
| 4 | 7.21 | 11.62 | Negative |
| 5 | 10.6 | 11.79 | Negative |
| 6 | 16.5 | 11.98 | Negative |
| 7 | 25.3 | 12.16 | Negative |
| 8 | 40.53 | 12.37 | Negative |
| 9 | 65.34 | 12.58 | Negative |
| 10 | 100.01 | 12.76 | Positive |
| 11 | 149.52 | 12.93 | Positive |
| 12 | 199.04 | 13.06 | Positive |

From these results, the virus retention capacity was 12.58 Log[copies/m$^2$]. This value was smaller by 0.36 than the virus retention capacity (12.94) of Planova 20N measured using the FE mixed liquid (1)-E. Also, the value was smaller by 0.35 than the average virus retention capacity (12.93) of Planova 20N measured using the viral capsid-containing liquids that contained no empty viral particle. These results indicate that the virus removal capability of a virus filter is underestimated in capability evaluation using a viral capsid-containing liquid that contains empty particles.

[Comparative Example 2]

The FE mixed liquid (2) shown in Table 2 was added to a biological material-containing solution (pH 4.5) containing 10 mg/mL (final concentration) human globulin and 0.1 mol/L NaCl (a total of 100 mL), and this solution was filtered through Planova 20N (manufactured by Asahi Kasei Medical Co., Ltd., membrane area: 0.001 m$^2$). The number of MVM nucleic acid copies per mL of the biological material-containing solution supplemented with the FE mixed liquid (2) was 7.85 Log$_{10}$[copies/mL]. The filtration conditions involved 78.4 kPa and 25°C. The collection of fractions and the quantification of a viral nucleic acid were carried out in the same manner as the method described in Example 1 except that the FE mixed liquid (2) was used. The filtration results are shown in Table 10.

[Table 10]

| Fraction No. [A] | Total filtration volume (mL) [B] | Total amount of virus load per unit membrane area (Log [copies/m$^2$]) [C] | Virus detection [D] |
|---|---|---|---|
| 1 | 1.64 | 11.06 | Negative |
| 2 | 2.65 | 11.27 | Negative |
| 3 | 4.23 | 11.47 | Negative |
| 4 | 6.92 | 11.69 | Negative |
| 5 | 10.54 | 11.87 | Negative |
| 6 | 16.72 | 12.07 | Negative |
| 7 | 25.56 | 12.26 | Negative |
| 8 | 40.55 | 12.46 | Negative |
| 9 | 66.89 | 12.67 | Positive |
| 10 | 104.51 | 12.87 | Positive |

From these results, the virus retention capacity was 12.46 Log[copies/m$^2$]. This value was smaller by 0.46 than the virus retention capacity (12.92) of Planova 20N measured using the FE mixed liquid (2)-E. Also, the value was smaller by 0.47 than the average virus retention capacity (12.93) of Planova 20N measured using the viral capsid-containing liquids that contained no empty viral particle. These results indicate that the virus removal capability of a virus filter is underestimated in capability evaluation using a viral capsid-containing liquid that contains empty particles.

[Comparative Example 3]

The FE mixed liquid (3) shown in Table 2 was added to a biological material-containing solution (pH 4.5) containing 10 mg/mL (final concentration) human globulin and 0.1 mol/L NaCl (a total of 100 mL), and this solution was filtered through Planova 20N (manufactured by Asahi Kasei Medical Co., Ltd., membrane area: 0.001 m$^2$). The number of MVM nucleic acid copies per mL of the biological material-containing solution supplemented with the FE mixed liquid (2) was 7.89 Log$_{10}$[copies/mL]. The filtration conditions involved 78.4 kPa and 25°C. The collection of fractions and the quantification of a viral nucleic acid were carried out in the same manner as the method described in Example 1 except that the FE mixed liquid (3) was used. The filtration results are shown in Table 11.

[Table 11]

| Fraction No. [A] | Total filtration volume (mL) [B] | Total amount of virus load per unit membrane area (Log [copies/m$^2$]) [C] | Virus detection [D] |
|---|---|---|---|
| 1 | 1.57 | 11.09 | Negative |
| 2 | 2.56 | 11.30 | Negative |
| 3 | 4.05 | 11.50 | Negative |
| 4 | 6.55 | 11.71 | Negative |

(continued)

| Fraction No. [A] | Total filtration volume (mL) [B] | Total amount of virus load per unit membrane area (Log [copies/m$^2$]) [C] | Virus detection [D] |
|---|---|---|---|
| 5 | 9.95 | 11.89 | Positive |
| 6 | 16.68 | 12.11 | Positive |
| 7 | 25.06 | 12.29 | Positive |
| 8 | 40.48 | 12.50 | Positive |
| 9 | 65.84 | 12.71 | Positive |
| 10 | 100.37 | 12.89 | Positive |

From these results, the virus retention capacity was 11.71 Log[copies/m$^2$]. This value was smaller by 1.23 than the virus retention capacity (12.94) of Planova 20N measured using the FE mixed liquid (3)-E. Also, the value was smaller by 1.22 than the average virus retention capacity (12.93) of Planova 20N measured using the viral capsid-containing liquids that contained no empty viral particle. These results indicate that the virus removal capability of a virus filter is underestimated in capability evaluation using a viral capsid-containing liquid that contains empty particles.

**Claims**

1. A method for evaluating the viral clearance capability of a virus removal medium, comprising the steps of:

   (a) adding a viral capsid-containing liquid to a solution to be purified;
   (b) contacting the virus removal medium with the solution to be purified that has been supplemented with the viral capsid-containing liquid to harvest a purified solution; and
   (c) quantifying the number of total viral capsid in the solution to be purified before the purification and the number of total viral capsid in the purified solution.

2. The method according to claim 1, wherein in the step (b), the solution to be purified that has been supplemented with the viral capsid-containing liquid is passed through the virus removal medium to harvest the solution purified.

3. The method according to claim 1 or 2, wherein

   empty viral particles have been removed from the viral capsid-containing liquid to be added to the solution to be purified, and
   in the step (c), a nucleic acid of a virus in the solution to be purified before the purification and a nucleic acid of a virus in the purified solution are quantified.

4. The method according to claim 3, wherein the empty viral particles are removed by centrifugation.

5. The method according to claim 3 or 4, wherein the empty viral particles are removed by density gradient ultracentrifugation.

6. The method according to any one of claims 3 to 5, wherein the empty viral particles are removed by density gradient ultracentrifugation using iodixanol.

7. The method according to any one of claims 3 to 6, wherein the nucleic acid is quantified by quantitative PCR or fluorescence flow cytometry.

8. The method according to any one of claims 3 to 7, wherein the nucleic acid is quantified by quantitative PCR.

9. The method according to any one of claims 1 to 8, wherein the virus removal medium is a medium having a form of a membrane or beads.

10. The method according to any one of claims 1 to 9, wherein the virus removal medium is a virus removal membrane.

11. The method according to any one of claims 3 to 8, further comprising the step of removing a free nucleic acid in the solution before the step of quantifying the nucleic acid of the virus.

12. The method according to claim 11, wherein the free nucleic acid is removed with nuclease.

13. The method according to claim 12, wherein the nuclease is DNaseI or Benzonase.

14. The method according to any one of claims 1 to 13, wherein the virus is a virus belonging to *Parvoviridae.*

15. The method according to any one of claims 1 to 14, wherein the viral capsid-containing liquid is a viral capsid-containing liquid that contains a naturally derived viral capsid.

16. The method according to any one of claims 1 to 15, wherein the viral capsid-containing liquid contains viral particles having a nucleic acid, and empty viral particles.

# Fig. 1

MVM concentrated culture solution (uL)  2.5  5

Recombinant VP2 standard (ng)  2  5  10  20

VP2 —
VP3 —

Recombinant VP2 standard

## INTERNATIONAL SEARCH REPORT

| | International application No. |
|---|---|
| | PCT/JP2021/007566 |

**A. CLASSIFICATION OF SUBJECT MATTER**
C12Q 1/34(2006.01)i; C12Q 1/6851(2018.01)i; C12Q 1/686(2018.01)i; C12Q
1/70(2006.01)i; G01N 33/569(2006.01)i
FI: C12Q1/70; G01N33/569 G; C12Q1/6851 Z; C12Q1/686 Z; C12Q1/34
According to International Patent Classification (IPC) or to both national classification and IPC

**B. FIELDS SEARCHED**

Minimum documentation searched (classification system followed by classification symbols)
C12Q1/34; C12Q1/6851; C12Q1/686; C12Q1/70; G01N33/569

Documentation searched other than minimum documentation to the extent that such documents are included in the fields searched
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN);
PubMed

**C. DOCUMENTS CONSIDERED TO BE RELEVANT**

| Category* | Citation of document, with indication, where appropriate, of the relevant passages | Relevant to claim No. |
|---|---|---|
| X | JP 2016-529908 A (MOCKV SOLUTIONS INC.) 29 September 2016 (2016-09-29) claims, paragraphs [0001], [0018], [0024], examples 5-7 | 1, 2, 9, 10, 14-16 |
| A | | 3-8, 11-13 |
| A | JP 2014-526246 A (UNIQURE IP B.V.) 06 October 2014 (2014-10-06) paragraphs [0066]-[0069] | 3-8, 11-13 |
| A | JP 2013-523175 A (EMD MILLIPORE CORPORATION) 17 June 2013 (2013-06-17) claims | 3-8, 11-13 |
| A | JP 2019-537435 A (BAYER AG.) 26 December 2019 (2019-12-26) claims | 3-8, 11-13 |

☒ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 26 April 2021 (26.04.2021) | 11 May 2021 (11.05.2021) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2021/007566 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 2015-510765 A (EMD MILLIPORE CORP.) 13 April 2015 (2015-04-13) claims | 3-8, 11-13 |

Form PCT/ISA/210 (continuation of second sheet) (January 2015)

Form PCT/ISA/210 (patent family annex) (January 2015)

| INTERNATIONAL SEARCH REPORT<br>Information on patent family members | | International application No.<br>PCT/JP2021/007566 | |
|---|---|---|---|
| Patent Documents referred in the Report | Publication Date | Patent Family | Publication Date |
| JP 2016-529908 A | 29 Sep. 2016 | US 2015/0072339 A1<br>claims, paragraphs [0001], [0018], [0024], examples 5-7<br>WO 2015/036917 A2<br>EP 3044339 A2<br>CN 105899684 A | |
| JP 2014-526246 A | 06 Oct. 2014 | US 2014/0342434 A1<br>paragraphs [0038]-[0071]<br>WO 2013/036118 A1<br>EP 2744895 A1<br>CN 103857790 A<br>KR 10-2014-0074333 A | |
| JP 2013-523175 A | 17 Jun. 2013 | US 2012/0088228 A1<br>claims<br>WO 2011/130119 A2<br>EP 2377927 A1<br>CN 102985536 A<br>KR 10-2013-0020954 A | |
| JP 2019-537435 A | 26 Dec. 2019 | US 2019/0316213 A1<br>claims<br>WO 2018/075716 A1<br>EP 3141611 A2<br>KR 10-2019-0066017 A<br>CN 110100008 A | |
| JP 2015-510765 A | 13 Apr. 2015 | US 2015/0166965 A1<br>claims<br>WO 2013/138465 A1<br>EP 2825639 A1<br>CN 104160019 A | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014080676 A1
- US 20120088228
- JP 4024041 B
- JP 6220447 B

**Non-patent literature cited in the description**

- Guideline on safety assurance of fractionated plasma products against viruses. Pharmaceutical and Medical Safety Bureau, Ministry of Health, Labour and Welfare, 30 August 1999
- **RAPHAEL WOLFISBERG et al.** *Journal of Virology,* 2016
- **BEATRIZ MAROTO et al.** *JOURNAL OF VIROLOGY,* 2004
- **UIRUSU JIKKENGAKU KAKURON.** Virology Experiments in Detail in English. Maruzen Publishing Co., Ltd, 22-23
- **V. HUTORNOJS et al.** *Env, Exp. Biol.,* 2012, vol. 10, 117-123
- **ANTHONY M. D'ABRAMO JR. et al.** *Virology,* 2005
- **JOSHUA C GRIEGER et al.** *Molecular Therapy,* 2015
- **PAVEL PLEVKA et al.** *JOURNAL OF VIROLOGY,* 2011
- **PETER TATTERSALL et al.** *JOURNAL OF VIROLOGY,* 1976